# EUROPÄISCHE PATENTSCHRIFT

(11) **EP 4 354 209 B1**
(45) Veröffentlichungstag und Bekanntmachung des Hinweises auf die Patenterteilung: **20.05.2026**
(21) Anmeldenummer: 23196172.3
(22) Anmeldetag: 08.09.2023
(51) Int. Cl.: G02C 1/04, G02C 1/00, A61F 9/02

(54) **BRILLE**
EYEWEAR
LUNETTES

(30) Priorität: 13.10.2022 DE 102022210819
(43) Veröffentlichungstag der Anmeldung: 17.04.2024
(73) Patentinhaber: Uvex Arbeitsschutz GmbH, 90766 Fürth (DE)
(72) Erfinder: Wolf, Martin, 91522 Ansbach (DE); Kühnlein, Florian, 90587 Veitsbronn (DE); Abel, Michael, 90537 Feucht (DE); Schuss, Frederic, 90419 Nürnberg (DE)
(74) Vertreter: Rau, Schneck & Hübner Patentanwälte Rechtsanwälte PartGmbB

(56) Entgegenhaltungen:
- US-A- 5 815 235
- US-A1- 2004 141 147
- US-A1- 2005 036 104
- US-A1- 2006 279 692
- US-B1- 6 742 890
- US-B1- 6 939 004

## Beschreibung

Die Erfindung betrifft eine Brille, insbesondere Arbeitsschutzbrille, gemäß dem Oberbegriff des Anspruchs 1. Sie kann beispielsweise auch als Sportbrille, optische Brille oder dergleichen ausgebildet sein. Ferner richtet sich die Erfindung auf ein Verfahren zum Zusammensetzen bzw. Montieren einer derartigen Brille.

Brillen mit austauschbaren Einzelteilen sind aus dem Stand der Technik allgemein bekannt. Für das Zusammensetzen der Brillen sind häufig spezielle Hilfsmittel bzw. Werkzeuge erforderlich. Ferner haben die Einzelteile in der Regel komplizierte Geometrien.

Eine in der US 6 939 004 B1 offenbarte gattungsgemäße Brille hat einen Primärrahmen und einen austauschbaren Sekundärrahmen. Der Primärrahmen umfasst ein horizontales Stirnteil, das ein Nasenabstützelement trägt. In zusammengesetztem Zustand greift eine Eingriffsplatte des Sekundärrahmens in einen Eingriffsraum des Primärrahmens ein. Ferner greifen Vorsprünge, die benachbart zu einer Nasenöffnung an dem Sekundärrahmen angeordnet sind, in Eingriffsnuten des Nasenabstützelements ein. Die Montage dieser Brille ist oftmals beschwerlich.

Die US 2006/0279692 A1 offenbart eine Arbeitsschutzbrille mit einem oberen Rahmenteil, an dem Befestigungseinrichtungen für eine Scheibe ausgebildet sind.

Die US 2005/0036104 A1 offenbart eine Schutzbrille mit einer Brillenscheibe, die einfach und schnell entfernbar ist. Die Schutzbrille hat außerdem einen einstückigen Rahmen mit einem Stirnstrebenbereich sowie äu-ßeren Bügelbereichen. Die Brillenscheibe ist innenseitig oder außenseitig an dem Brillenrahmen befestigt.

Aus der US 5 815 235 sind Skibrillen mit verschwenkbaren oberen und unteren Rahmenelementen bekannt, die ein Entfernen und Austauschen einer Brillenscheibe erlauben. Die Rahmenelemente begrenzen eine Öffnung für die Brillenscheibe und sind zwischen einer Schließposition und Öffnungsposition verschwenkbar.

Die US 6 742 890 B1 offenbart eine Befestigungsanordnung für eine Scheibe und einen Rahmen einer Brille.

Eine aus der US 2004/0141147 A1 bekannte Brille umfasst eine einstückige Scheibe und einen Stirnrahmen sowie ein demontierbares Nasenstück, das ausgebildet ist, an dem Rahmen und der Scheibe anzugreifen.

Die US 2005/0 036 104 A1, US 5 815 235, US 2013/0 185 849 A1 und US 2006/0 279 692 A1 offenbaren weitere Brillen des Standes der Technik.

Der Erfindung liegt die Aufgabe zugrunde, eine Brille bereitzustellen, die zum einen vergleichsweise einfach fertigbar und zum anderen äußerst montagefreundlich ist.

Diese Aufgabe wird erfindungsgemäß durch die in den Hauptansprüchen 1 und 10 angegebenen Merkmale gelöst. Der Kern der Erfindung liegt in dem Zusammensetzen bzw. Montieren der Brillenscheibenanordnung und des Brillenrahmens durch Verschwenken derselben relativ zueinander um bzw. über mindestens, bevorzugt genau, eine Schwenkstelle. Die mindestens eine Schwenkstelle liefert beispielsweise mindestens eine, bevorzugt genau eine, Schwenkachse bzw. mindestens einen, bevorzugt genau einen, Schwenkbereich für das, insbesondere zumindest teilweise geführte, Zusammensetzen der Brillenscheibenanordnung und des Brillenrahmens.

Beispielsweise erfolgt für das Zusammensetzen der Brillenscheibenanordnung und des Brillenrahmens ein Verschwenken der Brillenscheibenanordnung gegenüber dem Brillenrahmen oder des Brillenrahmens gegenüber der Brillenscheibenanordnung. Zum Beispiel erfolgt auch ein gemeinsames Verschwenken der Brillenscheibenanordnung und des Brillenrahmens entsprechend zueinander.

Es ist zweckmäßig, wenn das Zusammensetzen der Brillenscheibenanordnung und des Brillenrahmens manuell und günstigerweise werkzeuglos, also ohne Hilfsmittel, möglich bzw. durchführbar ist. Es ist bevorzugt intuitiv durchführbar. Vorzugsweise ist es dafür vermeidbar, die Brillenscheibenanordnung in deren Sichtbereich zu berühren. Es ist von Vorteil, wenn separate Verbindungsmittel hinfällig sind.

Das mindestens eine untere Vorzentrierungs-Schwenkmittel und das mindestens eine untere Vorzentrierungs-Schwenkgegenmittel sorgen für das vorgegebene bzw. definierte Verschwenken der Brillenscheibenanordnung und des Brillenrahmens gegeneinander für das Zusammensetzen derselben. Sie sind bevorzugt zumindest in einem Wechselwirkungsbereich komplementär zueinander ausgebildet. Das mindestens eine untere Vorzentrierungs-Schwenkmittel ist vorzugsweise in einem seitlichen Rand- bzw. Endbereich der Brillenscheibenanordnung angeordnet. Es ist zweckmäßig, wenn ein unteres Vorzentrierungs-Schwenkmittel-Paar und ein unteres Vorzentrierungs-Schwenkgegenmittel-Paar vorhanden ist.

Das mindestens eine obere Vorzentrierungs-Mittel und das mindestens eine obere Vorzentrierungs-Gegenmittelsorgen sorgen für eine belastbare, einfach erzielbare Verbindung zwischen der Brillenscheibenanordnung und des Brillenrahmens. Sie sind zum Beispiel zumindest in einem Wechselwirkungsbereich komplementär zueinander ausgebildet. Das mindestens eine obere Vorzentrierungs-Mittel ist vorzugsweise in einem seitlichen Rand- bzw. Endbereich der Brillenscheibenanordnung angeordnet. Es ist zweckmäßig, wenn ein oberes Vorzentrierungs-Mittel-Paar und ein oberes Vorzentrierungs-Gegenmittel-Paar vorhanden ist.

Die Brille ist in zusammengesetztem Zustand der Brillenscheibenanordnung und des Brillenrahmens besonders stabil bzw. belastbar. Es ist zweckmäßig, wenn die Brillenscheibenanordnung und der Brillenrahmen in zusammengesetztem Zustand in direkter Verbindung miteinander stehen. Der Brillenrahmen umgreift die Brillenscheibenanordnung, insbesondere bereichsweise, von oben. Zwischen der Brillenscheibenanordnung und dem Brillenrahmen liegt zumindest bereichsweise eine Nut-Feder-Verbindung vor.

Die Brillenscheibenanordnung umfasst beispielsweise genau eine einstückige, durchgehende Brillenscheibe. Alternativ hat diese beispielsweise zwei Brillenscheiben. Die Brillenscheibenanordnung ist bevorzugt wechselbar.

Der Brillenrahmen ist bevorzugt imstande, die Brillenscheibenanordnung zu halten bzw. zu tragen, wenn die Brillenscheibenanordnung und der Brillenrahmen zusammengesetzt sind. Er umläuft dann günstigerweise zumindest teilweise die Brillenscheibenanordnung und hat vorzugsweise eine zumindest teilweise nach außen räumlich begrenzte Brillenscheibenanordnungs-Aufnahme zum Aufnehmen der Brillenscheibenanordnung. Es ist von Vorteil, wenn die Brillenscheibenanordnungs-Aufnahme nach unten offen ist. Günstigerweise ist der Brillenrahmen aus Kunststoff gebildet. Vorzugsweise ist er ein Spritzgussteil.

Es ist zweckmäßig, wenn die Brille eine Kopf-Halteeinrichtung zum Halten der Brillenscheibenanordnung an einem Brillenträger aufweist. Die Kopf-Halteeinrichtung ist bevorzugt an dem Brillenrahmen angeordnet. Sie ist zum Beispiel durch mindestens ein Halteband gebildet, das beim Tragen der Brille den Hinterkopf des Brillenträgers umläuft. Alternativ ist sie beispielsweise durch zwei, insbesondere verschwenkbare, Brillenbügel gebildet.

Vorzugsweise sind die Brillenscheibenanordnung und der Brillenrahmen wieder voneinander trennbar. Es ist zweckmäßig, wenn das Trennen der Brillenscheibenanordnung und des Brillenrahmens voneinander bzw. deren Demontage (im Wesentlichen) analog zu dem Zusammensetzen der Brillenscheibenanordnung und des Brillenrahmens, bevorzugt werkzeuglos, erfolgt.

Es ist von Vorteil, wenn die Brille bezüglich einer Hauptebene symmetrisch ist.

Eine Nasenabstützvorrichtung zur Abstützung des Brillenrahmens gegenüber einer Nase des Brillenträgers führt zu einem besonders hohen Tragekomfort. Es ist zweckmäßig, wenn sie zumindest bereichsweise nachgiebig ist. Die Nasenabstützvorrichtung ist bevorzugt integraler Bestandteil des Brillenrahmens.

Die Nasenabstützvorrichtungs-Aufnahme und die Nasenabstützvorrichtung sind bevorzugt komplementär zueinander ausgebildet. Die Nasenabstützvorrichtung ist vorzugsweise (im Wesentlichen) V- bzw. Y-förmig.

Weitere vorteilhafte Ausgestaltungen der Erfindung sind in den Unteransprüchen angegeben.

Die Ausgestaltung gemäß dem Unteranspruch 2 erlaubt ein kontrolliertes bzw. definiertes Verschwenken der Brillenscheibenanordnung und des Brillenrahmens für ein Zusammensetzen derselben gegeneinander. Das Zusammensetzen ist so besonders funktionssicher bzw. gut kontrollierbar durchführbar bzw. benutzerfreundlich.

Das mindestens eine untere Vorzentrierungs-Schwenkmittel ist gemäß dem Unteranspruch 3 als Aufnahme, insbesondere schlüssellochartige Aufnahme, insbesondere schlüssellochartige Rastaufnahme, ausgebildet. Die Aufnahme ist bevorzugt nach unten offen. Sie ist günstigerweise nach oben und seitlich begrenzt.

Das mindestens eine untere Vorzentrierungs-Schwenkgegenmittel ist bevorzugt als Körper ausgeführt und hat günstigerweise eine zylindrischen bzw. teilzylindrischen Querschnitt bzw. Lagerbereich. Der Körper ist beispielsweise als Stift, Ansatz oder dergleichen ausgebildet.

Das mindestens eine untere Vorzentrierungs-Schwenkmittel und das mindestens eine untere Vorzentrierungs-Schwenkgegenmittel bilden bevorzugt ein erstes Vorzentrierungs-Schwenkmittel bzw. Vorzentrierungs-Schwenkgegenmittel. Es ist zweckmäßig, wenn das untere Vorzentrierungs-Schwenkgegenmittel mit dem mindestens einen unteren Vorzentrierungs-Schwenkmittel bei dem Zusammensetzen der Brillenscheibenanordnung und des Brillenrahmens und in deren zusammengesetztem Zustand miteinander, insbesondere lösbar, in Rastverbindung stehen, was besonders montagefreundlich ist und zu einer besonders funktionssicheren Brille führt. Es ist von Vorteil, wenn das mindestens eine untere Vorzentrierungs-Schwenkmittel integraler Bestandteil der Brillenscheibenanordnung ist. Günstigerweise ist das mindestens eine untere Vorzentrierungs-Schwenkgegenmittel integraler Bestandteil des Brillenrahmens.

Das mindestens eine obere Vorzentrierungs-Mittel ist gemäß dem Unteranspruch 4 zum Beispiel als Steg, insbesondere Anlagesteg, Platte, Stift oder dergleichen ausgebildet.

Das mindestens eine obere Vorzentrierungs-Gegenmittel ist bevorzugt als Aufnahme, Anlagefläche, Anlageflanke oder dergleichen ausgeführt.

Das mindestens eine obere Vorzentrierungs-Mittel und das mindestens eine obere Vorzentrierungs-Gegenmittel bilden bevorzugt ein zweites Vorzentrierungs-Mittel bzw. Vorzentrierungs-Gegenmittel. Das mindestens eine obere Vorzentrierungs-Mittel bzw. Vorzentrierungs-Gegenmittel ist/sind bevorzugt bei dem Zusammensetzen der Brillenscheibenanordnung und des Brillenrahmens zeitlich nach dem mindestens einen unteren Vorzentrierungs-Schwenkmittel bzw. Vorzentrierungs-Schwenkgegenmittel aktiv bzw. wirksam.

Es ist von Vorteil, wenn das mindestens eine obere Vorzentrierungs-Mittel integraler Bestandteil der Brillenscheibenanordnung ist. Günstigerweise ist das mindestens eine obere Vorzentrierungs-Gegenmittel integraler Bestandteil des Brillenrahmens.

Eine Brille gemäß Unteranspruch 5 ist besonders einfach fertigbar und montagefreundlich.

Auch die Ausgestaltung gemäß dem Unteranspruch 6 führt zu einer Brille, die in zusammengesetztem Zustand der Brillenscheibenanordnung und des Brillenrahmens besonders stabil bzw. belastbar ist. Vorzugsweise liegt dann eine formschlüssige Verbindung zwischen der Nasenabstützvorrichtung und der Nasenabstützvorrichtungs-Aufnahme vor. Beispielsweise liegt die Nasenabstützvorrichtung unten, oben und/oder seitlich innen an der Brillenscheibenanordnung bzw. der Nasenabstützvorrichtungs-Aufnahme an bzw. stützt sich dort ab.

Die Ausbildung gemäß dem Unteranspruch 7 ergibt ebenfalls eine Brille, die besonders stabil bzw. belastbar ist. Es ist zweckmäßig, wenn die Nasenabstützvorrichtung, insbesondere selbständig, nach dem Verschwenken aus ihrer Ausgangsstellung bzw. Tragestellung in eine Montagestellung wieder in die Ausgangsstellung zurückschwenkt bzw. zurückfedert.

Die Nasenabstützvorrichtung gemäß dem Unteranspruch 8 ist zum Beispiel nach vorne oben verschwenkbar.

Das Rahmenteil gemäß dem Unteranspruch 9 ist zumindest bereichsweise tordierbar bzw. in sich, bevorzugt um seine Längsmittelachse, verdrehbar. Es ist dort vorzugsweise zumindest bereichsweise elastisch.

Die hier verwendeten Ausdrücke "oben", "unten", "vorne", "hinten", "rückseitig", "seitlich" oder dergleichen beziehen sich insbesondere auf eine Orientierung der Brille bei einem ordnungsgemäßen Tragen derselben durch einen Brillenträger.

Nachfolgend wird unter Bezugnahme auf die beigefügte Zeichnung eine bevorzugte Ausführungsform der Erfindung beispielhaft beschrieben. Dabei zeigen:
- Fig. 1: eine perspektivische Ansicht einer erfindungsgemäßen Brille in deren vollständig zusammengesetztem Zustand bzw. Tragestellung,

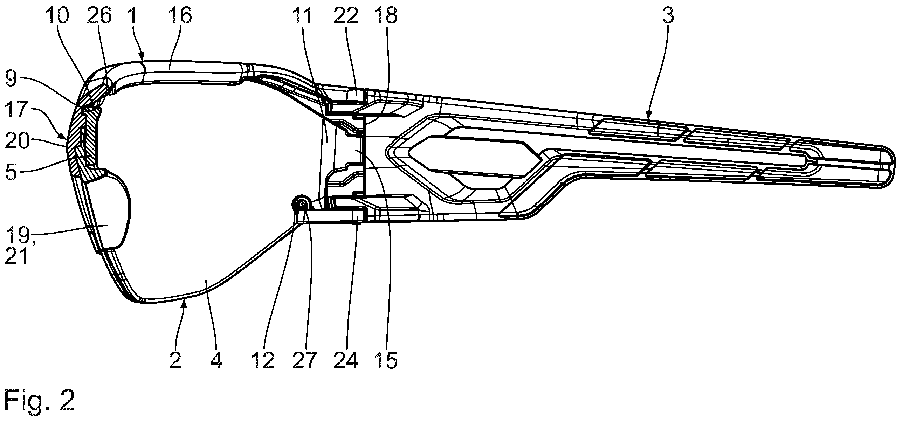

- Fig. 2: einen Schnitt durch die in Fig. 1 veranschaulichte Brille, der durch deren Nasenbereich geht,
- Fig. 3: einen Schnitt durch die in Fig. 1 dargestellte Brille, der seitlich versetzt zu dem Schnitt gemäß Fig. 2 ist,

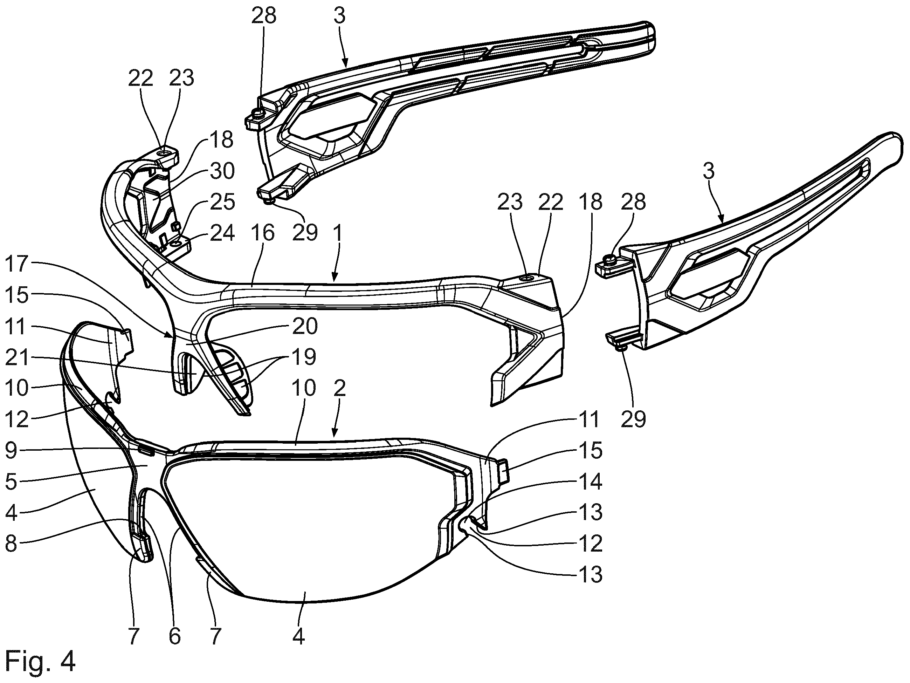

- Fig. 4: eine Explosionsansicht der in Fig. 1 gezeigten Brille,
- Fig. 5: eine rückseitige Ansicht des Brillenrahmens der in Fig. 1 gezeigten Brille, und
- Fig. 6 bis 8: Ansichten, die das Zusammensetzen des Brillenrahmens und der Brillenscheibenanordnung veranschaulichen

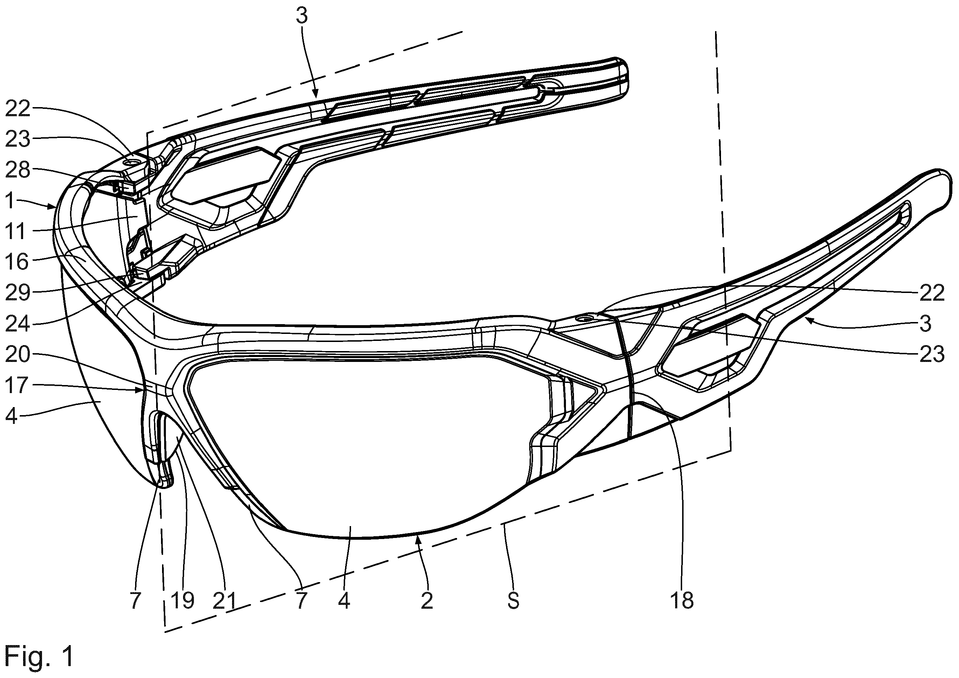

Eine in Fig. 1 in ihrer Gesamtheit, in vollständig zusammengesetztem Zustand veranschaulichte Brille umfasst einen Brillenrahmen 1, eine von dem Brillenrahmen 1 gehaltene Brillenscheibenanordnung 2 und zwei Brillenbügel 3, die an dem Brillenrahmen 1 endseitig angelenkt sind. Die Brille ist bezüglich einer Symmetrieebene S symmetrisch, die durch deren Nasenbereich geht.

Die Brillenscheibenanordnung 2 ist einteilig und symmetrisch ausgebildet. Sie umfasst zwei nebeneinander angeordnete Brillenscheiben 4 und einen Verbindungssteg 5, der zwischen den Brillenscheiben 4 verläuft und diese oben bogenartig miteinander verbindet. Die Brillenscheibenanordnung 2 ist gekrümmt.

Die Brillenscheibenanordnung 2 hat außerdem zwei Anschlussarme 6, die sich unten an den Verbindungssteg 5 anschließen. Die Anschlussarme 6 verlaufen beabstandet zueinander und erstrecken sich von dem Verbindungssteg 5 aus nach unten. Sie verlaufen von dem Verbindungssteg 5 aus voneinander weg. Jeder Anschlussarm 6 erstreckt sich benachbart zu einer Brillenscheibe 4.

Jeder Anschlussarm 6 trägt unten einen in Richtung auf die Symmetrieebene S vorspringenden Rückhalteansatz bzw. Rückhaltevorsprung bzw. Rückhalteabsatz 7, der eine nach oben bzw. dem Verbindungssteg 5 zugewandte Rückhaltefläche 8 ausbildet.

Der Verbindungssteg 5, die Anschlussarme 6 und die Rückhalteansätze 7 bilden bzw. begrenzen eine Nasenabstützvorrichtungs-Aufnahme.

Der Verbindungssteg 5 trägt oben vorne einen Rastkörper 9, der nach vorne vorspringt und länglich ist. Er erstreckt sich senkrecht zu der Symmetrieebene S und ist beispielsweise quaderförmig oder stegförmig ausgebildet.

Die Brillenscheibenanordnung 2 umfasst ferner oben zwei Abschlusselemente 10. Jedes Abschlusselement 10 ist zum Beispiel stegartig ausgebildet und schließt sich oben an eine Brillenscheibe 4 an. Jedes Abschlusselement 10 verläuft von dem Verbindungssteg 5 nach seitlich außen.

Außerdem weist die Brillenscheibenanordnung 2 zwei Seitenelemente 11 auf, die sich seitlich außen an das jeweilige Abschlusselement 10 und die jeweilige Brillenscheibe 4 anschließen.

In jedem Seitenelement 11 ist unten eine schlüssellochartige Aufnahme 12 ausgebildet, die durchgängig und nach unten offen ist. Jede Aufnahme 12 ist durch zwei einander gegenüberliegende Einführflanken 13 begrenzt, die von unten nach oben aufeinander zu laufen. Jede Aufnahme 12 weist außerdem einen inneren Schwenklagerbereich 14 auf, der sich an die beiden Einführflanken 13 anschließt und im Querschnitt teilkreisförmig bzw. kreisförmig ist. Jeder Schwenklagerbereich 14 hat eine maximale Quererstreckung, die (geringfügig) größer als der benachbarte (minimale) Abstand der Einführflanken 13 zueinander ist. Jede Aufnahme 12 bildet eine erste bzw. untere Vorzentrierungs-Schwenkaufnahme.

Jedes Seitenelement 11 weist außerdem ein Vorzentrierungs-Mittel 15 auf, das oberhalb der benachbarten Aufnahme 12 angeordnet ist. Jedes Vorzentrierungs-Mittel 15 ist randseitig an dem jeweiligen Seitenelement 11 angeordnet und springt nach hinten vor. Jedes Vorzentrierungs-Mittel 15 bildet ein zweites bzw. oberes Vorzentrierungs-Mittel. Jedes Vorzentrierungs-Mittel 15 ist als Überstand ausgebildet und zum Beispiel stegartig, plattenförmig, stiftartig oder dergleichen.

Der Brillenrahmen 1 ist einstückig und symmetrisch. Er erstreckt sich gekrümmt.

Der Brillenrahmen 1 umfasst ein oberes längliches Rahmenteil 16 und eine von dem oberen Rahmenteil 16 mittig nach unten vorspringende Nasenabstützvorrichtung 17. Ferner hat der Brillenrahmen 1 zwei seitliche Endelemente 18, die sich seitlich außen an das jeweilige Rahmenteil 16 anschließen.

Das Rahmenteil 16 ist zumindest bereichsweise elastisch bzw. flexibel ausgebildet. Insbesondere ist es bei Aufbringung einer äußeren Kraft um seine Längsmittelachse in einer Umfangsrichtung U tordierbar, so dass die Nasenabstützvorrichtung 17 gegenüber den Endelementen 18 nach oben aus ihrer Tragestellung (Fig. 1) verschwenkbar ist. Das Rahmenteil 16 hat zum Beispiel einen eckigen und/oder runden Querschnitt.

Die Nasenabstützvorrichtung 17 umfasst zwei benachbart zueinander angeordnete Nasenauflagestege 19 und einen diese miteinander verbindenden Verbindungskörper 20. Die Nasenauflagestege 19 sind länglich und verlaufen von dem Verbindungskörper 20 nach unten. Sie laufen von dem Verbindungskörper 20 aus voneinander weg. Jeder Nasenauflagesteg 19 hat innen eine Nasenauflagefläche 21, die beim Tragen der Brille durch einen Brillenträger (nicht dargestellt) auf dessen Nase aufliegt.

Jedes Endelement 18 trägt einen oberen Anlenkansatz 22 mit einer oberen zylindrischen Lageraussparung 23 und einen unteren Anlenkansatz 24 mit einer unteren zylindrischen Lageraussparung 25. Die Lageraussparungen 23, 25 von jedem Anlenkansatz 22, 24 sind übereinander angeordnet und geben eine Anlenkachse für den jeweiligen Brillenbügel 3 vor.

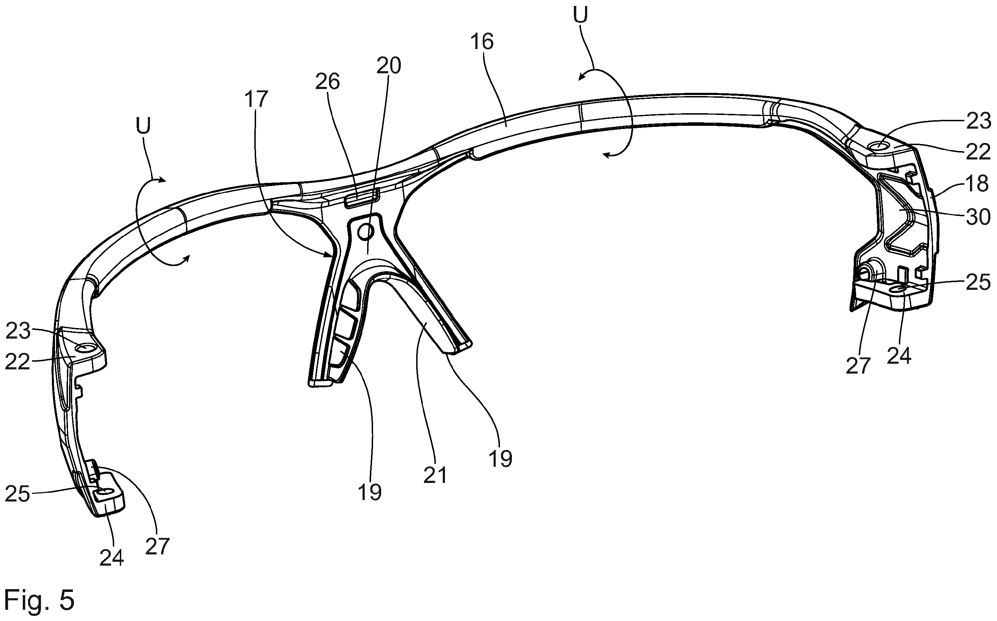

Jedes Endelement 18 trägt ferner einen Vorzentrierungs-Stift 27 (Fig. 5), der im Querschnitt teilkreisförmig oder kreisförmig ist. Jeder Vorzentrierungs-Stift 27 ist oberhalb des benachbarten unteren Anlenkansatzes 24 angeordnet. Beispielsweise schließt sich jeder Vorzentrierungs-Stift 27 an den benachbarten unteren Anlenkansatz 24 oben direkt an. Die Vorzentrierungs-Stifte 27 sind innenseitig an den Endelementen 18 angeordnet. Sie bilden untere bzw. erste Vorzentrierungs-Stifte 27.

Jedes Endelement 18 hat außerdem ein Vorzentrierungs-Anlagemittel 30, das oberhalb des benachbarten Vorzentrierungs-Stifts 27 ausgebildet ist. Jedes Vorzentrierungs-Anlagemittel 30 ist innenseitig an dem jeweiligen Endelement 18 und zum Beispiel endseitig oder beabstandet zu einem äu-ßeren Ende des jeweiligen Endelements 18 angeordnet. Die Vorzentrierungs-Anlagemittel 30 sind durch eine Innenseite bzw. Innenflanke des jeweiligen Endelements 18 gebildet. Sie sind zum Beispiel nach unten, oben, innen und/oder außen offen bzw. begrenzt. Sie bilden obere bzw. zweite Vorzentrierungs-Anlagemittel 30.

Der Brillenrahmen 1 trägt außerdem rückseitig mittig eine längliche Rastaussparung 26 (Fig. 5), die komplementär zu dem Rastkörper 9 ausgebildet ist.

Jeder Brillenbügel 3 weist einen oberen Lagerkörper 28 und einen unteren Lagerkörper 29 auf. Die Lagerkörper 28, 29 sind jeweils zapfen- bzw. stiftförmig. Sie sind an jedem Brillenbügel 3 übereinander angeordnet und springen zum Beispiel voneinander weg. Die oberen Lagerkörper 28 sind komplementär zu den oberen Lageraussparungen 23 ausgebildet, während die unteren Lagerkörper 29 komplementär zu den unteren Lageraussparungen 25 sind. Eine andere Anordnung der Lagerkörper 28, 29 und Lageraussparungen 23, 25 ist alternativ möglich. Zum Beispiel hat jeder Brillenbügel 3 nur genau einen Lagerkörper 28 bzw. 29.

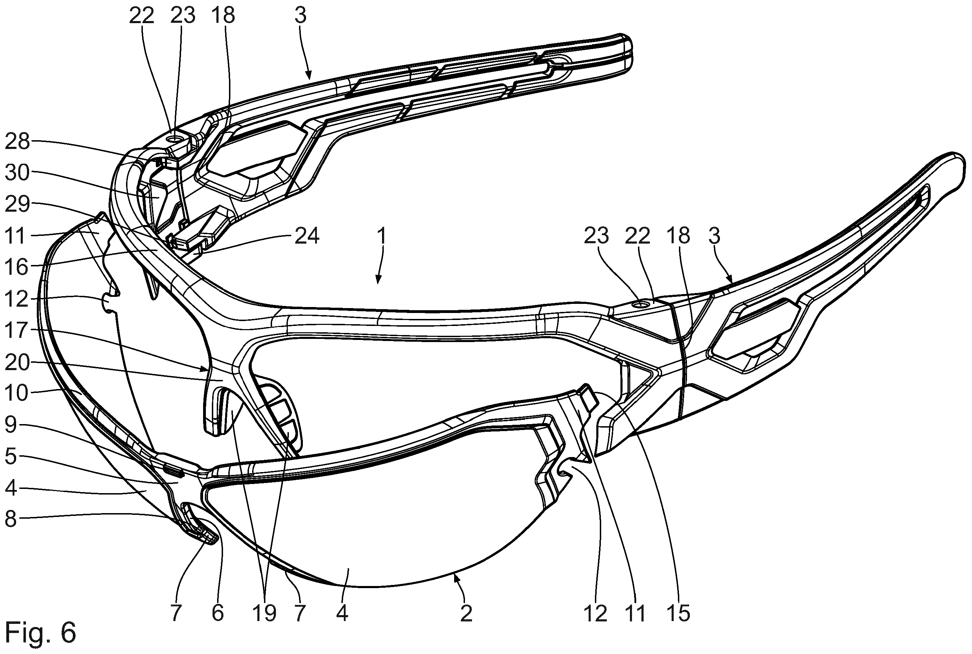

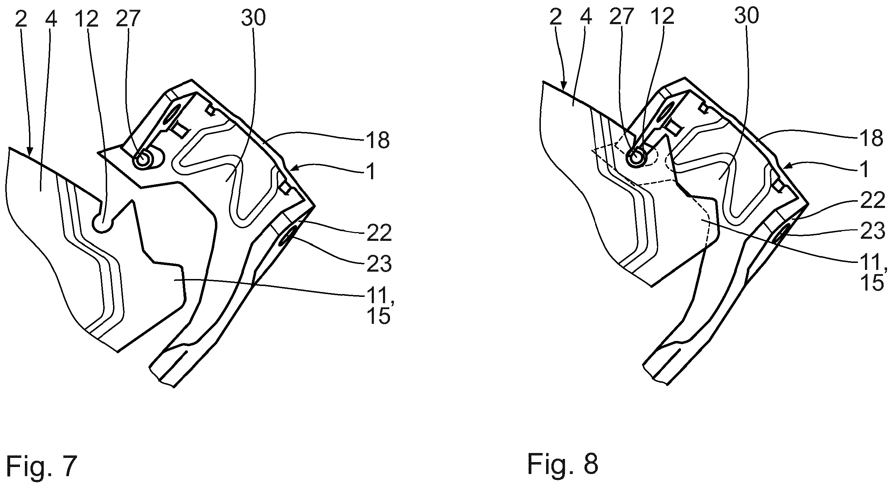

Nachfolgend wird auch unter Bezugnahme auf Fig. 6 bis 8 das Zusammensetzen des Brillenrahmens 1 und der Brillenscheibenanordnung 2 sowie der Brillenbügel 3, das heißt deren Montage, beschrieben.

Der Brillenrahmen 1, die Brillenscheibenanordnung 2 und die Brillenbügel 3 sind ursprünglich separat.

Zunächst werden die Vorzentrierungs-Stifte 27, bevorzugt gleichzeitig bzw. gleichmäßig, von unten über entsprechende Einführöffnungen der Aufnahmen 12 und die Einführflanken 13 in die Schwenklagerbereiche 14 geführt. Alternativ oder zusätzlich werden die Aufnahmen 12 gegenüber den Vorzentrierungs-Stiften 27 entsprechend bewegt.

Die Vorzentrierungs-Stifte 27 drücken dabei die Einführflanken 13 zeitweise voneinander weg und rasten bzw. schnappen in den jeweiligen Schwenklagerbereich 14 ein. Alternativ oder zusätzlich sind die Vorzentrierungs-Stifte 27 zusammendrückbar, um in den jeweiligen Schwenklagerbereich 14 zu gelangen. Jeder Schwenklagerbereich 14 liefert eine Teil-Schwenkachse und bildet ein Schwenklager für den jeweiligen Vorzentrierungs-Stift 27 aus. Die Teil-Schwenkachsen fallen (im Wesentlichen) zusammen und liefern eine gemeinsame Schwenkachse.

Anschließend werden der Brillenrahmen 1 und die Brillenscheibenanordnung 2 um die durch die Schwenklagerbereiche 14 vorgegebene gemeinsame horizontale untere Schwenkachse über einen entsprechend vorgegebenen Schwenkweg relativ zueinander kontrolliert bzw. geführt verschwenkt, sodass sich der Verbindungssteg 5 und die Nasenabstützvorrichtung 17 einander nähern. Bei der Näherung befindet sich die Brillenscheibenanordnung 2 vor der Nasenabstützvorrichtung 17. Eine andere Ausgestaltung bzw. Ausbildung der Brillenscheibenanordnung 2 und/oder Nasenabstützvorrichtung 17 ist alternativ möglich.

Anschließend wird die Nasenabstützvorrichtung 17 nach vorne oben manuell verschwenkt, was aufgrund des tordierbaren Rahmenteils 16 möglich ist. Die Brillenscheibenanordnung 2 ist so imstande, unter der Nasenabstützvorrichtung 17 an dieser vorbeigeschwenkt zu werden.

Nach dem Passieren der Nasenabstützvorrichtung 17 schwenkt die Nasenabstützvorrichtung 17 wieder nach unten in ihre Tragestellung. Dies kann manuell erfolgen. Die Nasenabstützvorrichtung 17 kann jedoch auch eigenständig aufgrund ihres Rückfedervermögens in ihre Tragestellung zurückschwenken.

Die Vorzentrierungs-Mittel 15 werden innenseitig an den Endelementen 18 entlanggeführt und gelangen zu den Vorzentrierungs-Anlagemittel 30, wo diese, insbesondere flächig, anliegen.

Der Rastkörper 9 rastet in die Rastaussparung 26 ein.

Die Nasenabstützvorrichtung 17 greift in die Nasenabstützvorrichtungs-Aufnahme ein (Fig. 1, 5).

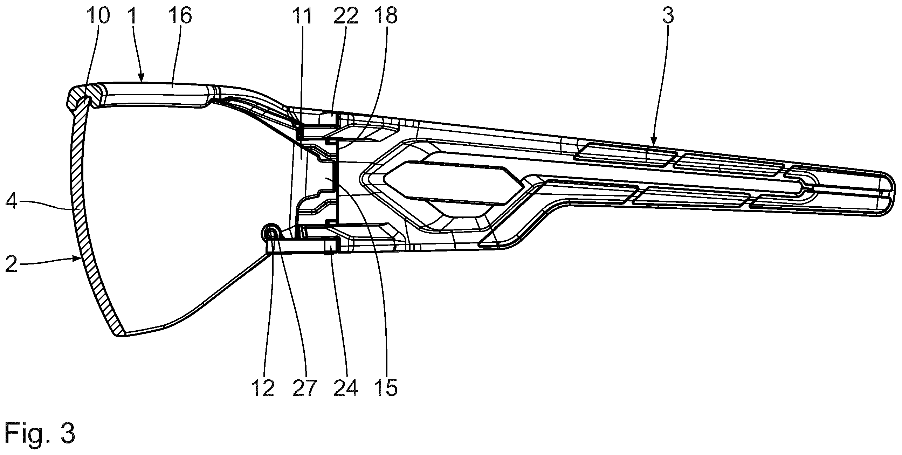

Die Abschlusselemente 10 greifen von unten in das Rahmenteil 16 ein (Fig. 3).

Anschließend wird von jedem Brillenbügel 3 der obere Lagerkörper 28 von unten in die obere Lageraussparung 23 und der untere Lagerkörper 29 von oben in die untere Lageraussparung 25 eingeführt, sodass der Brillenbügel 3 entsprechend an dem Brillenrahmen 1 angelenkt ist.

Im fertigem zusammengesetztem Zustand schließen sich die Nasenauflagestege 19 innenseitig an die Anschlussarme 6 und bevorzugt auch unten an den Verbindungssteg 5 an. Jeder Nasenauflagesteg 19 stützt sich fußseitig gegenüber der Rückhaltefläche 8 des jeweiligen Rückhalteansatzes 7 ab.

Durch den Eingriff des Rastkörpers 9 in die Rastaussparung 26 ist die Brillenscheibenanordnung 2 nach oben, unten und seitlich sowie bevorzugt nach vorne gegenüber dem Brillenrahmen 1 fixiert. Durch den Eingriff der Abstützelemente 10 von unten in das Rahmenteil 16 ist die Brillenscheibenanordnung 2 nach vorne, hinten, oben und zum Beispiel auch seitlich gegenüber dem Brillenrahmen 1 fixiert. Durch den Eingriff der Nasenabstützvorrichtung 17 in die Nasenabstützvorrichtungs-Aufnahme ist die Brillenscheibenanordnung 2 seitlich, nach unten und bevorzugt nach oben gegenüber dem Brillenrahmen 1 fixiert. Durch die Anlage der Vorzentrierungs-Mittel 15 innenseitig an den Endelementen 18 ist die Brillenscheibenanordnung 2 seitlich gegenüber dem Brillenrahmen 1 fixiert. Der Brillenrahmen 1 und die Brillenscheibenanordnung 2 sind in zusammengesetztem Zustand miteinander verspannt. Sie sind lösbar miteinander verbunden.

Beim Tragen der Brille liegen die Nasenauflageflächen 21 flächig an der Nase des Brillenträgers an. Die Nasenauflagestege 19 sorgen für ein bequemes Tragen der Brille und einen sicheren Halt. Die Brillenscheiben 4 befinden sich vor den Augen des Brillenträgers. Die Brillenbügel 3 liegen seitlich an dem Kopf des Brillenträgers an.

Eine Demontage erfolgt im Wesentlichen analog. Auch dabei erfolgt ein kontrolliertes Verschwenken des Brillenrahmens 1 und der Brillenscheibenanordnung 2 zueinander.

## Patentansprüche

1. Brille,
a) mit einer Brillenscheibenanordnung (2),
b) mit einem Brillenrahmen (1), und
c) mit mindestens einer Schwenkstelle für ein Zusammensetzen der Brillenscheibenanordnung (2) und des Brillenrahmens (1) durch Verschwenken derselben gegeneinander,
d) wobei der Brillenrahmen (1) eine Nasenabstützvorrichtung (17) zur Abstützung gegenüber einer Nase eines Brillenträgers aufweist, und
e) wobei die Brillenscheibenanordnung (2) eine Nasenabstützvorrichtungs-Aufnahme aufweist, die in zusammengesetztem Zustand der Brillenscheibenanordnung (2) und des Brillenrahmens (1) die Nasenabstützvorrichtung (17) aufnimmt,
**dadurch gekennzeichnet, dass**
f) die Brillenscheibenanordnung (2) mindestens ein unteres Vorzentrierungs-Schwenkmittel (12) und der Brillenrahmen (1) mindestens ein unteres Vorzentrierungs-Schwenkgegenmittel (27) zum Wechselwirken mit dem mindestens einen unteren Vorzentrierungs-Schwenkmittel (12) bei dem Zusammensetzen der Brillenscheibenanordnung (2) und des Brillenrahmens (1) aufweist,
g) die Brillenscheibenanordnung (2) mindestens ein oberes Vorzentrierungs-Mittel (15) und der Brillenrahmen (1) mindestens ein oberes Vorzentrierungs-Gegenmittel (30) zum Wechselwirken mit dem mindestens einen oberen Vorzentrierungs-Mittel (15) bei dem Zusammensetzen der Brillenscheibenanordnung (2) und des Brillenrahmens (1) aufweist und
h) die Brillenscheibenanordnung (2) und der Brillenrahmen (1) in zusammengesetztem Zustand in mindestens einem oberen Brillenbereich derart in formschlüssiger Verbindung miteinander stehen, dass der Brillenrahmen (1) die Brillenscheibenanordnung (2) von oben umgreift, wobei zwischen der Brillenscheibenanordnung (2) und dem Brillenrahmen (1) eine Nut-Feder-Verbindung vorliegt.

2. Brille nach Anspruch 1, **dadurch gekennzeichnet, dass** die mindestens eine Schwenkstelle vorgegeben ist.

3. Brille nach Anspruch 1 oder 2, **dadurch gekennzeichnet, dass** das mindestens eine untere Vorzentrierungs-Schwenkmittel (12) als Aufnahme, insbesondere schlüssellochartige Aufnahme, ausgebildet ist.

4. Brille nach einem der vorherigen Ansprüche, **dadurch gekennzeichnet, dass** das mindestens eine obere Vorzentrierungs-Mittel (15) als Überstand ausgebildet ist.

5. Brille nach einem der vorherigen Ansprüche, **dadurch gekennzeichnet, dass** das mindestens eine obere Vorzentrierungs-Mittel (15) in einem seitlichen Randbereich der Brillenscheibenanordnung (2) angeordnet ist.

6. Brille nach einem der vorherigen Ansprüche, **dadurch gekennzeichnet, dass** die Nasenabstützvorrichtung (17) und die Nasenabstützvorrichtungs-Aufnahme in zusammengesetztem Zustand der Brillenscheibenanordnung (2) und des Brillenrahmens (1) in riegelartiger Verbindung miteinander stehen.

7. Brille nach einem der vorherigen Ansprüche, **dadurch gekennzeichnet, dass** die Nasenabstützvorrichtung (17) zum Zusammensetzen der Brillenscheibenanordnung (2) und des Brillenrahmens (1) in ihrer Gesamtheit verschwenkbar ist.

8. Brille nach einem der vorherigen Ansprüche, **dadurch gekennzeichnet, dass** die Nasenabstützvorrichtung (17) nach oben verschwenkbar ist.

9. Brille nach Anspruch 7 oder 8, **dadurch gekennzeichnet, dass** der Brillenrahmen (1) ein die Nasenabstützvorrichtung (17) tragendes Rahmenteil (16) aufweist, das zur Verschwenkung der Nasenabstützvorrichtung (17) zumindest bereichsweise tordierbar ist.

10. Verfahren zum Zusammensetzen der Brille nach einem der vorherigen Ansprüche, umfassend den Schritt
- Verschwenken der Brillenscheibenanordnung (2) und des Brillenrahmens (1) gegeneinander um mindestens eine Schwenkstelle.

11. Verfahren nach Anspruch 10, **dadurch gekennzeichnet, dass** die Nasenabstützvorrichtung (17) für das Verschwenken der Brillenscheibenanordnung (2) und des Brillenrahmens (1) gegeneinander, insbesondere nach oben, verschwenkt wird.

## Claims

1. Glasses,
a) having a lens arrangement (2),
b) having a glasses frame (1), and
c) having at least one pivot point for joining together the lens arrangement (2) and the glasses frame (1) by pivoting them relative to one another,
d) wherein the glasses frame (1) has a nose support device (17) for support against a nose of a glasses wearer, and
e) wherein the lens arrangement (2) has a nose support device receptacle which, in a joined-together state of the lens arrangement (2) and the glasses frame (1), receives the nose support device (17),
**characterized in that**
f) the lens arrangement (2) has at least one lower pre-centring pivoting means (12), and the glasses frame (1) has at least one lower counterpart pre-centring pivoting means (27) for interacting with the at least one lower pre-centring pivoting means (12) upon joining together the lens arrangement (2) and the glasses frame (1).
g) the lens arrangement (2) has at least one upper pre-centring means (15), and the glasses frame (1) has at least one upper counterpart pre-centring means (30) for interacting with the at least one upper pre-centring means (15) upon joining together the lens arrangement (2) and the glasses frame (1) and
h) the lens arrangement (2) and the glasses frame (1) are connected to one another by positive engagement in at least one upper glasses region in the joined-together state, such that the glasses frame (1) engages around the lens arrangement (2) from above, wherein a tongue-and-groove connection is present between the lens arrangement (2) and the glasses frame (1).

2. Glasses according to Claim 1, **characterized in that** the at least one pivot point is predefined.

3. Glasses according to Claim 1 or 2, **characterized in that** the at least one lower pre-centring pivoting means (12) is designed as a receptacle, in particular a keyhole-like receptacle.

4. Glasses according to one of the preceding claims, **characterized in that** the at least one upper pre-centring means (15) is designed as a protrusion.

5. Glasses according to any one of the preceding claims, **characterized in that** the at least one upper pre-centring pivoting means (15) is arranged in a lateral edge region of the lens arrangement (2).

6. Glasses according to any one of the preceding claims, **characterized in that** the nose support device (17) and the nose support device receptacle are connected to one another in a bolt-like manner in the joined-together state of the lens arrangement (2) and the glasses frame (1).

7. Glasses according to any one of the preceding claims, **characterized in that** the nose support device (17) is pivotable as a whole for joining together of the lens arrangement (2) and the glasses frame (1).

8. Glasses according to any one of the preceding claims, **characterized in that** the nose support device (17) is pivotable upwards.

9. Glasses according to Claim 7 or 8, **characterized in that** the glasses frame (1) has a frame part (16) which carries the nose support device (17) and is twistable, at least partially, in order to pivot the nose support device (17).

10. Method for joining together the glasses according to any one of the preceding claims, comprising the step of
- pivoting the lens arrangement (2) and the glasses frame (1) relative to one another about at least one pivot point.

11. Method according to Claim 10, **characterized in that** the nose support device (17) is pivoted, in particular upwards, for the pivoting of the lens arrangement (2) and the glasses frame (1) relative to one another.

## Revendications

1. Lunettes,
a) comportant un ensemble verres de lunettes (2),
b) comportant un cadre de lunettes (1), et
c) comportant au moins un point de pivotement pour un assemblage de l'ensemble verres de lunettes (2) et du cadre de lunettes (1) par pivotement de ceux-ci l'un par rapport à l'autre,
d) dans lesquelles le cadre de lunettes (1) présente un dispositif d'appui sur le nez (17) destiné à l'appui par rapport à un nez d'un porteur de lunettes, et
e) dans lesquelles l'ensemble verres de lunettes (2) présente un logement de dispositif d'appui sur le nez qui, lorsque l'ensemble verres de lunettes (2) et le cadre de lunettes (1) sont assemblés, loge le dispositif d'appui sur le nez (17),
**caractérisées en ce que**
f) l'ensemble verres de lunettes (2) présente au moins un moyen de pivotement de précentrage inférieur (12) et le cadre de lunettes (1) présente au moins un moyen complémentaire de pivotement de précentrage inférieur (27) destiné à interagir avec l'au moins un moyen de pivotement de précentrage inférieur (12) lors de l'assemblage de l'ensemble verres de lunettes (2) et du cadre de lunettes (1),
g) l'ensemble verres de lunettes (2) présente au moins un moyen de précentrage supérieur (15) et le cadre de lunettes (1) présente au moins un moyen complémentaire de précentrage supérieur (30) destiné à interagir avec l'au moins un moyen de précentrage supérieur (15) lors de l'assemblage de l'ensemble verres de lunettes (2) et du cadre de lunettes (1) et
h) l'ensemble verres de lunettes (2) et le cadre de lunettes (1), une fois assemblés, sont reliés l'un à l'autre par complémentarité de forme dans au moins une zone de lunettes supérieure de telle sorte que le cadre de lunettes (1) enserre l'ensemble verres de lunettes (2) par le haut, une liaison à rainure et languette se trouvant entre l'ensemble verres de lunettes (2) et le cadre de lunettes (1).

2. Lunettes selon la revendication 1, **caractérisées en ce que** l'au moins un point de pivotement est prédéfini.

3. Lunettes selon la revendication 1 ou 2, **caractérisées en ce que** l'au moins un moyen de pivotement de précentrage inférieur (12) est réalisé sous la forme d'un logement, notamment un logement de type trou de serrure.

4. Lunettes selon l'une des revendications précédentes, **caractérisées en ce que** l'au moins un moyen de précentrage supérieur (15) est réalisé sous la forme d'une saillie.

5. Lunettes selon l'une des revendications précédentes, **caractérisées en ce que** l'au moins un moyen de précentrage supérieur (15) est disposé dans une zone de bord latérale de l'ensemble verres de lunettes (2).

6. Lunettes selon l'une des revendications précédentes, **caractérisées en ce que** le dispositif d'appui sur le nez (17) et le logement de dispositif d'appui sur le nez sont, lorsque l'ensemble verres de lunettes (2) et le cadre de lunettes (1) sont assemblés, reliés l'un à l'autre à la manière d'un verrou.

7. Lunettes selon l'une des revendications précédentes, **caractérisées en ce que** le dispositif d'appui sur le nez (17) peut être pivoté dans son ensemble pour assembler l'ensemble verres de lunettes (2) et le cadre de lunettes (1).

8. Lunettes selon l'une des revendications précédentes, **caractérisées en ce que** le dispositif d'appui sur le nez (17) peut être pivoté vers le haut.

9. Lunettes selon la revendication 7 ou 8, **caractérisées en ce que** le cadre de lunettes (1) présente une partie de cadre (16) portant le dispositif d'appui sur le nez (17), qui peut être tordue au moins par régions pour le pivotement du dispositif d'appui sur le nez (17).

10. Procédé d'assemblage des lunettes selon l'une des revendications précédentes, comprenant l'étape consistant à
- pivoter l'ensemble verres de lunettes (2) et le cadre de lunettes (1) l'un par rapport à l'autre autour d'au moins un point de pivotement.

11. Procédé selon la revendication 10, **caractérisé en ce que** le dispositif d'appui sur le nez (17) est pivoté, notamment vers le haut, pour le pivotement de l'ensemble verres de lunettes (2) et du cadre de lunettes (1) l'un par rapport à l'autre.
